# FASCICULE DE BREVET EUROPEEN

(11) **EP 3 687 988 B1**
(45) Date de publication et mention de la délivrance du brevet: **03.05.2023**
(21) Numéro de dépôt: 18783063.3
(22) Date de dépôt: 24.09.2018
(51) Int. Cl.: C07D 403/12, A01N 43/713, C12Q 1/04, C12N 1/20

(54) **COMPOSE ANTIBACTERIEN, ET APPLICATIONS**
ANTIBAKTERIELLE VERBINDUNG UND VERWENDUNGEN DAVON
ANTIBACTERIAL COMPOUND AND USES OF SAME

(30) Priorité: 25.09.2017 FR 1758830
(43) Date de publication de la demande: 05.08.2020
(73) Titulaire: bioMérieux, 69280 Marcy-l'Étoile (FR)
(72) Inventeur: GRAY, Mark, Woodstone Village County Durham DH4 6TX (GB); KONDACS, Laszlo, Sunderland SR2 7NX (GB); ORENGA, Sylvain, 01160 Neuville Sur Ain (FR); PERRY, John, High Heaton Newcastle Upon Tyne NE7 7BH (GB)
(74) Mandataire: bioMérieux PI Groupement mandataires
(86) Numéro de dépôt international: PCT/FR2018/052321
(87) Numéro de publication internationale: WO 2019/058074

(56) Documents cités:
- WO-A1-02/22785
- ARROYO G ET AL: "Selective action of inhibitors used in different culture media on the competitive microflora of Salmonella", JOURNAL OF APPLIED BACTERIOLOGY, BLACKWELL PUBLISHING LTD., OXFORD, GB, vol. 78, no. 3, 1 janvier 1995 (1995-01-01), pages 281-289, XP002555076, ISSN: 0021-8847
- LENDA ET AL: "Synthesis of new tetrazole and triazole substituted pyroglutamic acid and proline derivatives", TETRAHEDRON LETTERS, ELSEVIER, AMSTERDAM , NL, vol. 48, no. 5, 4 January 2007 (2007-01-04), pages 805-808, XP005821590, ISSN: 0040-4039, DOI: 10.1016/J.TETLET.2006.11.163

## Description

L'invention concerne le domaine de la microbiologie, et plus particulièrement les domaines du diagnostic clinique *in vitro* et du contrôle sanitaire microbiologique. Elle a pour objet un nouveau composé antibactérien et vise l'application de celui-ci comme agent sélectif dans des méthodes et des milieux de culture microbiologique destinés à la détection, l'identification, l'enrichissement, le dénombrement et/ou l'isolement de microorganismes cibles susceptibles d'être présents dans un échantillon à analyser et/ou à traiter.

Les techniques de diagnostic *in vitro* ou de contrôle sanitaire, qui visent à mettre en évidence d'éventuelles infections ou contaminations microbiologiques, reposent encore sur des étapes de culture microbiologique permettant d'amplifier, détecter, identifier, dénombrer et/ou isoler les microorganismes d'intérêt éventuellement présents dans les échantillons à analyser. Parce que les microorganismes d'intérêt (ou microorganismes cibles) sont rarement les seuls présents dans les échantillons à analyser, et sont même bien souvent très minoritaires par rapport à la flore microbienne annexe (ou microorganismes non-cibles), il est généralement nécessaire d'utiliser des milieux de culture dits sélectifs, dont les compositions sont spécifiquement choisies pour optimiser la croissance et la multiplication du(des) microorganisme(s) cible(s), tout en bloquant autant que possible celles de la flore microbienne annexe.

Aussi, outre de devoir répondre aux exigences nutritives des microorganismes cibles et de créer un environnement et des conditions propices à ces microorganismes cibles, la composition des milieux de culture sélectifs renferment également des agents sélectifs aptes à ralentir ou à bloquer la pousse (c'est-à-dire la croissance, le développement et/ou la division) des microorganismes non-cibles.

De nombreux agents sélectifs aux propriétés antibactériennes ont été développés et implémentés dans les compositions de milieu de culture microbiologique. Chaque agent sélectif présente un spectre d'inhibition particulier, plus ou moins large, et une activité d'inhibition plus ou moins marquée selon le genre et/ou l'espèce de la bactérie considérée.

Le Tableau 1 ci-après liste quelques-uns des agents pathogènes les plus fréquemment criblés à partir d'échantillons alimentaires, d'eau, cliniques et environnementaux. Il expose également les agents sélectifs fréquemment utilisés dans les milieux de culture dédiés à leur détection, identification, enrichissement, dénombrement et/ou isolement de microorganismes, ainsi que quelques composés connus pour leur effet inhibiteur à leur égard.

**Tableau 1**

| Espèces bactériennes | Agents sélectifs | Composés inhibiteurs |
|---|---|---|
| *Acinetobacter baumannii* | Cefsulodine, sels biliaires, vancomycine | Colistine, gentamicine, polymixine |
| *Burkholderia cepacia* | Cétrimide, polymixine B, vancomycine | Ceftazidime, gentamicine |
| *Enterobacter cloacae* | Vancomycine, désoxycholate de sodium, sels biliaires | Aztréonam, colistine, polymixine |
| *Escherichia coli* | Vancomycine, désoxycholate de sodium, sels biliaires | Aztréonam, alafosfalin, colistine, gentamicine, polymixine |
| *Klebsiella pneumoniae* | Vancomycine, désoxycholate de sodium, sels biliaires | Aztréonam, colistine, gentamicine, polymixine |
| *Providencia rettgeri* | Vancomycine, désoxycholate de sodium, sels biliaires | Aztréonam |
| *Pseudomonas aeruginosa* | Vancomycine, désoxycholate de sodium, sels biliaires, cétrimide | Cefsulodine, gentamicine |
| *Salmonella* Typhimurium | Vancomycine, désoxycholate de sodium, sels biliaires, vert brillant | Aztréonam, colistine, gentamicine, polymixine |
| *Serratia marcescens* | Vancomycine, désoxycholate de sodium, sels biliaires | Aztreonam, gentamicine |
| *Yersinia enterocolitica* | Cefsulodine, irgasan, novobiocine | Aztreonam, colistine, gentamicine, polymixine |
| *Salmonella* Enteritidis | Vancomycine, sels biliaires, vert brillant | Aztréonam, colistine, gentamicine, polymixine |
| *Bacillus subtilis* | Acrylonitrile, polymixine, streptomycine | Désoxycholate de sodium, sels biliaires, cristal violet, vert brillant, vancomycine |
| *Enterococcus faecalis* | Azide de sodium, colistine, sels biliaires | Chlorure de lithium, vancomycine |
| *Enterococcus faecium* | Azide de sodium, colistine, sels biliaires | Chlorure de lithium, vancomycine |
| *Listeria monocytogenes* | Chlorure de lithium, colistine, polymixine | Vancomycine, gentamicine |
| *Staphylococcus epidermidis* | Acide nalidixique, aztréonam, colistine | Vancomycine, gentamicine |
| *Staphylococcus aureus* MSSA | Chlorure de sodium, chlorure de lithium, colistine | Vancomycine, méticilline, céfoxitine, gentamicine |
| *Staphylococcus aureus* MRSA | Chlorure de sodium, chlorure de lithium, méticilline, céfoxitine, colistine | Vancomycine |
| *Streptococcus agalactiae* | Acide nalidixique, aztrénonam, colistine | Vancomycine, sels biliaires |

WO02/22785 décrit une méthode pour l'enrichissement sélectif de bactéries, notamment de bactéries Gram négatives, utilisant notamment l'acide L-pyroglutamyl-L-1-aminoéthylphosphonique et l'acide L-pyroglutamyl-L-1-aminoéthylsulfonique.

ARROYO G ET AL: "Sélective action of inhibitors used in différent culture média on the compétitive microflora of Salmonella" JOURNAL OF APPLIED BACTERIOLOGY, BLACKWELL PUBLISHING LTD., OXFORD, GB, vol. 78, no. 3, 1 janvier 1995 (1995-01-01), pages 281-289 compare différents agents sélectifs pour la différentiation cellulaire structurellement très différents du L-pyroglutamyl-L-1-aminoéthyltétrazole.

Face à la très grande diversité de microorganismes qui nous entourent, à la diversité des échantillons faisant l'objet de tests diagnostic ou de contrôle sanitaire, et à celle des populations microbiennes présentes dans ces échantillons, il est avantageux de pouvoir disposer de la plus grande gamme possible d'agents sélectifs.

A cet égard, l'invention propose un nouveau composé aux propriétés antibactériennes particulièrement intéressantes et ayant pour formule développée : 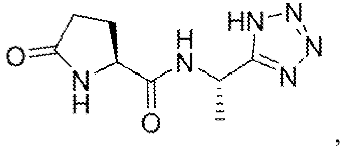 le L-pyroglutamyl-L-1-aminoéthyltétrazole.

L'invention s'étend également à ce composé sous forme de sels.

En utilisant la nomenclature IUPAC, ce composé peut aussi être désigné par : *N*-1-(1*H*-tétrazol-5-yl)éthyl)-5-oxopyrrolidine-2-carboxamide.

Avant d'aller plus avant dans la description de l'invention, les définitions suivantes sont données pour permettre une meilleure compréhension de celle-ci et de son intérêt pour le domaine de la microbiologie, en particulier pour le contrôle microbiologique.

Par « composé antibactérien », on entend un composé inhibiteur de bactéries. Cette activité inhibitrice peut se traduire soit par un effet bactéricide qui détruit des bactéries, soit un effet bactériostatique, qui bloque la croissance et/ou le développement des bactéries sans pour autant les tuer. A noter qu'un composé antibactérien peut être bactériostatique à faible dose et bactéricide à une dose plus élevée.

Par « milieu de culture microbiologique » ou plus simplement par « milieu de culture », on entend une préparation utilisée comme support à la culture de microorganismes, dont la composition comprend tous les éléments nécessaires à l'expression d'un métabolisme et/ou la survie et/ou la croissance des microorganismes ciblés. Un milieu de culture peut être liquide, solide, semi-solide. Par « milieu solide » ou « milieu semi-solide », on entend par exemple un milieu gélifié. L'agar est l'agent gélifiant traditionnel en microbiologie pour la préparation de ces milieux de culture, mais il est possible d'utiliser de la gélatine, de l'agarose ou d'autres gélifiants naturels ou artificiels. Le caractère « solide » ou « semi-solide » des milieux dépend essentiellement de la teneur en agent gélifiant. A des fins de simplification, on utilisera par la suite l'expression « milieu solide » pour désigner aussi bien un milieu solide qu'un milieu semi-solide.

Un milieu de culture est dit « sélectif » lorsqu'il comprend au moins un agent sélectif permettant audit milieu de favoriser la croissance d'un microorganisme cible ou d'un groupe cible de microorganismes, plutôt que celle de la flore microbienne annexe (composés de microorganismes non-cibles). Il s'agit essentiellement de composés antibactériens et/ou antifongiques, et présentant une spécificité de toxicité (toxicité plus faible pour les microorganismes cibles que les microorganismes non-cibles).

Par « agent sélectif », on entend tout composé susceptible d'inhiber la croissance et/ou le développement d'un microorganisme dit « non-cible ». A titre indicatif, un agent sélectif est utilisé, dans les milieux de culture sélectifs , à des concentrations comprises entre 0,001 et 1024,0 mg/L. A noter que le spectre d'inhibition d'un agent sélectif peut s'élargir avec l'augmentation de concentration.

Au sens de la présente invention, on entend par « diagnostic clinique *in vitro* » ou par « contrôle (sanitaire) microbiologique », l'analyse d'un échantillon dans le but de détecter et/ou de dénombrer des microorganismes suspectés/susceptibles d'être présents au sein dudit échantillon.

Par « échantillon », on entend une petite partie ou petite quantité de matière que l'on isole de son contexte à des fins de traitement et/ou d'analyse. Un échantillon peut consister en un prélèvement clinique d'origine humaine ou animale (en particulier, un prélèvement sanguin, de selles, d'urine, de muqueuses...). Il peut aussi avoir une origine industrielle ou environnementale, et consister en un prélèvement d'air, un prélèvement d'eau, un prélèvement effectué sur une surface, une pièce ou un produit manufacturé, un produit d'origine alimentaire (par exemple, un produit lacté, de la viande, du poisson, des oeufs, des fruits/légumes, une boisson...).

Par « détection », on entend la visualisation à l'oeil nu ou à l'aide d'un appareil optique de la présence du(es) microorganisme(s) cible(s), ou bien la mise en évidence de la présence du(es) microorganisme(s) cible(s) par la mise en oeuvre de techniques de biologie moléculaire, d'immunologie, de spectrométrie (notamment spectrométrie de masse, spectrométrie infrarouge...), de chromatographie.

Par « identification », on entend la détermination du genre et/ou de l'espèce et/ou un groupe au(x)quel(s) et/ou à laquelle appartient un microorganisme étudié.

Par « dénombrement », on entend le fait de comptabiliser/quantifier/évaluer le nombre de microorganismes cibles présents dans un échantillon testé.

Par « enrichissement », on entend le fait d'augmenter, dans une population microbienne d'un échantillon, la proportion que représente le(s) microorganisme(s) cible(s) par rapport à la population microbienne totale.

Par ses propriétés antibactériennes particulières, le L-pyroglutamyl-L-1-aminoéthyltétrazole suscite un intérêt tout particulier pour le domaine de l'analyse microbiologique. Au-delà du composé en lui-même, l'invention s'étend à l'utilisation *in vitro* du L-pyroglutamyl-L-1-aminoéthyltétrazole à titre de composé antibactérien et en particulier dans des méthodes et des milieux de culture microbiologique destinés à la détection, l'identification, l'enrichissement, le dénombrement et/ou l'isolement de microorganismes.

En particulier, l'invention concerne plus particulièrement une méthode de culture microbiologique *in vitro,* dans laquelle des microorganismes susceptibles d'être présents dans un échantillon à analyser et/ou à traiter sont ensemencés dans ou sur un milieu de culture microbiologique comprenant du L-pyroglutamyl-L-1-aminoéthyltétrazole, à une concentration non nulle, égale ou inférieure à 1 g/L.

Outre une activité antibactérienne *in vitro* marquée à l'égard d'espèces bactériennes spécifiques, le nouveau composé selon l'invention a démontré une grande modularité de son activité antibactérienne en fonction de la concentration utilisée.

Aussi, dans une méthode de culture microbiologique selon l'invention, le L-pyroglutamyl-L-1-aminoéthyltétrazole est avantageusement présent dans le milieu de culture microbiologique à une concentration :
- comprise entre 10 et 1000 mg/L, de préférence entre 16 et 500 mg/L, typiquement entre 30 et 128 mg/L, pour inhiber au moins une bactérie choisie parmi les espèces bactériennes consistant en : *Klebsiella pneumoniae, Serratia marcescens, Yersinia enterocolitica, Bacillus subtilis, Enterococcus faecalis* et *Enterococcus faecium* ; ou
- comprise entre 2 et 50 mg/L, de préférence entre 5 et 15 mg/L, pour inhiber au moins une bactérie choisie parmi les espèces bactériennes consistant en : *Klebsiella pneumoniae, Serratia marcescens, Yersinia enterocolitica, Enterococcus faecalis* et *Enterococcus faecium* ; ou
- comprise entre 1 et 10 mg/L, de préférence entre 3 et 5 mg/L, pour inhiber au moins une bactérie choisie parmi les espèces bactériennes consistant en : *Yersinia enterocolitica, Enterococcus faecalis* et *Enterococcus faecium* ; ou
- non nulle, égale ou inférieure à 5 mg/L, de préférence égale ou inférieure à 3 mg./L pour inhiber au moins une bactérie choisie parmi les espèces bactériennes consistant en : *Enterococcus faecalis* et *Enterococcus faecium ;*
ladite au moins une bactérie étant susceptible d'être présente dans l'échantillon à analyser et/ou à traiter.

De même, dans une méthode de culture microbiologique selon l'invention, le L-pyroglutamyl-L-1-aminoéthyltétrazole est avantageusement présent dans le milieu de culture microbiologique à une concentration :
- comprise entre 10 et 1000 mg/L, de préférence entre 16 et 500 mg/L, et typiquement entre 30 et 128 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Listeria monocytogenes, Staphylococcus epidermidis, Staphylococcus aureus, Streptococcus agalactiae* ; ou
- comprise entre 2 et 50 mg/L, de préférence entre 5 et 15 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis, Listeria monocytogenes, Staphylococcus epidermidis, Staphylococcus aureus, Streptococcus agalactiae* ; ou
- comprise entre 1 et 10 mg/L, de préférence entre 3 et 5 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae* ; ou
- non nulle, égale ou inférieure à 5 mg/L, de préférence égale ou inférieure à 3 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae* ; ou
ledit au moins un microorganisme cible étant susceptible d'être présent dans l'échantillon à analyser et/ou à traiter.

Un autre objet de l'invention concerne des milieux de culture microbiologique comprenant, à titre d'agent sélectif, au moins du L-pyroglutamyl-L-1-aminoéthyltétrazole, à une concentration non nulle, égale ou inférieure à 1 g/L, et de préférence une concentration non nulle, égale ou inférieure à 500 mg/L.

Selon un premier mode de réalisation avantageux, un milieu de culture microbiologique selon l'invention comprend, à titre d'agent sélectif, au moins du L-pyroglutamyl-L-1-aminoéthyltétrazole, à une concentration comprise entre 10 et 1000 mg/L, de préférence entre 16 et 500 mg/L, et typiquement entre 30 et 128 mg/L. Un tel milieu de culture est adapté à la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae.*

Selon un deuxième mode de réalisation avantageux, un milieu de culture microbiologique selon l'invention comprend, à titre d'agent sélectif, au moins du L-pyroglutamyl-L-1-aminoéthyltétrazole, à une concentration comprise entre 2 et 50 mg/L, de préférence entre 5 et 15 mg/L. Un tel milieu de culture est adapté à la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis*, *Listeria monocytogenes, Staphylococcus epidermidis, Staphylococcus aureus, Streptococcus agalactiae.*

Selon un troisième mode de réalisation avantageux, un milieu de culture microbiologique selon l'invention, comprend, à titre d'agent sélectif, au moins du L-pyroglutamyl-L-1-aminoéthyltétrazole, à une concentration non nulle, égale ou inférieure à 5 mg/L, de préférence égale ou inférieure à 3 mg/L. Un tel milieu de culture est adapté à la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae.*

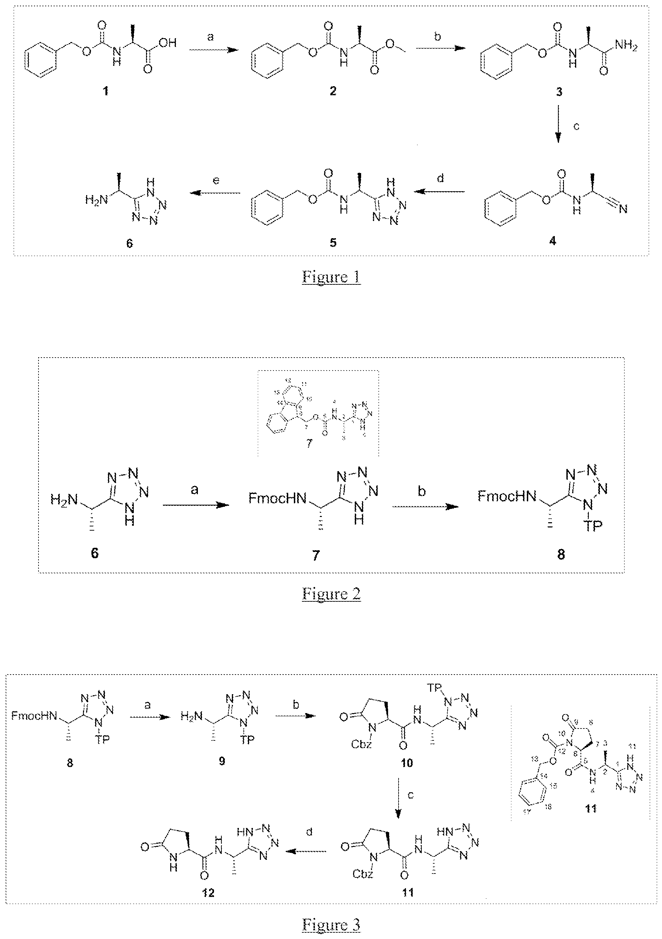

D'autres buts, caractéristiques et avantages de l'invention apparaîtront au vu de la description qui va suivre et des exemples développés ci-après. Les Figures 1 à 3, référencées dans cette description et ces exemples, présentent de manière schématique le déroulé d'un procédé de synthèse du L-pyroglutamyl-L-1-aminoéthyltétrazole.

Ces exemples ont pour but de faciliter la compréhension de l'invention, sa mise en oeuvre et son utilisation. Ces exemples sont donnés à titre explicatif et ne sauraient limiter la portée de l'invention.

### EXEMPLES

### EXEMPLE 1 : Synthèse des composés d'intérêt et données analytiques

### 1.1. Généralité

La synthèse des composés d'intérêt pour la mise en oeuvre de la présente invention est illustrée ci-après, en référence aux Figures 1 à 3.

Le suivi de ces synthèses et la caractérisation des produits finis et intermédiaires ont été réalisés par diverses méthodes d'analyse et notamment par spectroscopie par résonnance magnétique, spectroscopie infrarouge, spectrométrie de masse, détermination des points de fusion, chromatographie sur couche mince.

Les spectres RMN sont obtenus en utilisant un spectromètre Bruker Ultrashield 300 (à 300 MHz pour les spectres ¹H et à 75 MHz pour les spectres ¹³C), et un spectromètre Bruker Avance III Ultrashield (à 500 MHz pour les spectres ¹H et à 125 MHz pour les spectres ¹³C). Les points de fusion T_{f} sont enregistrés au moyen d'un microscope à platine chauffante Reichart-Kofler (les valeurs exposées sont non corrigés). Les spectres IR sont enregistrés sur une plage de 4000-600 cm⁻¹, en utilisant un spectrophotomètre Perkin Elmer Spectrum BX FT-IR. Les spectres de masse à faible résolution ont été enregistrés sur un analyseur Bruker Esquire 3000 Plus en utilisant la source d'électro-vaporisation, soit en mode ion positif soit en mode ion négatif. Les analyses élémentaires ont été effectuées à l'aide d'un Exeter Analytical CE-440 Elemental. Les chromatographies sur couche mince sont réalisées sur des gels de silice Merck 60F254. Des gels de silice 60 (35-70 µm) de Fisons sont utilisés pour réaliser les chromatographies flash ; les échantillons sont pré-absorbés sur la silice 60 (35-70 µm). Des réactions d'hydrogénation sont effectuées avec un mini réacteur distribué par la compagnie PARR, le 4560 Mini Bench Top Reactor.

Seulement une partie des données recueillies est présentée ci-après et ne concerne que des composés jugés importants pour la compréhension et la définition de la présente invention.

### 1.2. L-1-aminoéthyltétrazole (6)

### Synthèse :

La synthèse de la L-1-aminoéthyltétrazole (6) est réalisée en suivant la méthode de synthèse décrite par Bavetsias et al., 2000 (J. Med. Chem. 2000, 43, 1910) et à laquelle des modifications mineures ont été apportées. Le schéma réactionnel correspondant est présenté à la Figure 1.

A l'étape (a), la Cbz-L-alanine (1) est méthylée avec de l'iodure de méthyle en présence de Cs₂CO₃ dans du DMF.

Pour ce faire, à une solution de Cbz-L-alanine (1) (10,0 g ; 45,0 mmol) dans du DMF anhydre (70 mL), du carbonate de césium (8,03 g ; 25,0 mmol) est ajouté. L'ensemble est agité pendant 30 minutes à température ambiante, puis agité de nouveau pendant une nuit après addition d'iodure de méthyle (2,94 mL, 47,0 mmol), au goutte à goutte. Une fois la réaction terminée, de l'acétate d'éthyle (50 mL) est ajouté et le mélange est lavé à l'eau (3 x 50 mL), la phase aqueuse combinée est alors extraite avec de l'acétate d'éthyle (50 mL). La phase organique combinée est lavée avec une solution de K₂CO₃ 10 % (2 x 30 mL), avec de la saumure (30 mL), puis séchée sur MgSO₄. Le solvant est éliminé sous pression réduite pour donner un ester méthylique, le méthyl-(2S)-2-{[(benzyloxy)carbonyl]amino}propanoate ou ester méthylique de Cbz-L-alanine (2), se présentant sous la forme d'un solide blanc (10,6 g ; 99%).

L'ester méthylique de Cbz-L-alanine (2) est ensuite transformé, à l'étape (b), en un amide, le benzyl-N-[(1S)-1-carbamoyléthyl]carbamate (3). A cet effet, cet ester méthylique (2) (10,00 g ; 420 mmol) est dissout et agité dans une solution d'ammoniaque 7 M et de méthanol (60 ml), à 50°C et dans un tube scellé, pendant une nuit. Une fois la réaction terminée, le mélange est évaporé sous pression réduite pour donner le benzyl-N-[(1S)-1-carbamoyléthyl]carbamate ou Cbz-L-alanine amide (3), qui se présente sous la forme d'une poudre blanche (9,40 g ; 99%).

L'étape (c) permet de convertir le Cbz-L-alanine amide (3) en benzyl-N-[(1S)-1-cyanoethyl]carbamate ou Cbz-L-alanyl nitrile (4). Pour ce faire, du Cbz-L-alanine amide (3) (9,70 g ; 44,0 mmol) est dissout dans du DCM (21 mL), et de la pyridine (33 ml) y est ajoutée. Après refroidissement jusqu'à 0°C, du chlorure de tosyle (16,72 g ; 88,0 mmol) est ajouté, et le tout est agité pendant 30 minutes à 0°C puis pendant une nuit à température ambiante. Une fois la réaction terminée, le système est refroidi à 0°C, et bloqué avec de l'eau (160 mL) et de l'acétate d'éthyle (160 mL). Après séparation, la couche aqueuse est extraite à l'acétate d'éthyle (4 x 80 mL) et la phase organique combinée est lavée à l'HCl 1,2 M (3 x 80 mL) et au NaHCO₃ saturé (80 mL), puis à la saumure (80 mL). La solution totale est ensuite séchée sur MgSO₄ et évaporée sous pression réduite. Le produit brut est lavé à l'heptane pour donner le composé (4). Ce composé (4) se présente sous la forme de cristaux blancs (8,48 g ; 94%).

L'étape (d) permet de convertir le Cbz-L-alanyl nitrile (4) en benzyl-N-[(1S)-1-(1H-1,2,3,4-tétrazol-5-yl)ethyl]carbamate ou Cbz-L-1-aminoéthyltétrazole (5), par une réaction de cycloaddition dipolaire avec le NaN₃. Pour ce faire, du NH₄Cl (2,03 g ; 38 mmol) et du NaN₃ (2,62 g ; 38 mmoles) sont mis en suspension dans du DMF anhydre (60 mL), à l'intérieur d'un ballon à fond rond à deux cols, équipé d'un condenseur à reflux et d'un tube de séchage. Le Cbz-L-alanyl nitrile (4) y est ajouté (7,80 g, 38 mmoles). Le mélange a été agité et chauffé à 90°C pendant 1 heure. Au mélange refroidi, une autre portion de NH₄Cl (1,04 g ; 19 mmoles) et de NaN₃ (1,31 g ; 19 mmoles) est ajouté et le mélange est chauffé à 90°C pendant une nuit. Une fois la réaction terminée, le mélange est filtré et le résidu est lavé à l'acétate d'éthyle. Le filtrat est évaporé sous pression réduite. De l'eau (180 mL) est ajoutée au résidu et l'ensemble est acidifié à un pH de 1 avec une solution aqueuse d'HCl 2,5 M. Le précipité est séparé par filtration et lavé à l'eau pour donner le Cbz-L-1-aminoéthyltétrazole (5), sous la forme d'un solide blanc (7,55 g ; 80 %) .

A l'étape (e), le L-1-aminoéthyltétrazole (6) est finalement obtenu en éliminant le groupe protecteur Cbz. Cette déprotection est obtenue par une réaction d'hydrogénation catalysée au palladium. Pour ce faire, une solution de Cbz-L-1-aminoéthyltétrazole (5) (0,75 g ; 3,0 mmol) dans du méthanol est préparée. 1 meq de palladium sur charbon actif (5 % Pd) y est ajouté. Le mélange est agité à température ambiante sous 2 bars de pression d'hydrogène pendant une nuit dans un autoclave. Une fois la réaction terminée, le mélange est filtré à travers de la célite. Après filtration, le résidu solide est lavé à l'éthanol et le filtrat est évaporé sous pression réduite. Le L-1-aminoéthyltétrazole (6) obtenu se présente sous la forme d'un solide blanc (0,33 g ; 97%).

Le rendement global de cette synthèse de L-1-aminoéthyltétrazole (6) est de l'ordre de 71 %.
Caractérisation : 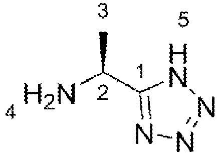 (1*S*)-1-(1*H*-1,2,3,4-tetrazol-5-yl)éthan-1-amine T_{f}=230°C
¹H NMR (300 MHz, DMSO-*d*₆) δ_{H} 4.55 (1H, quart, J=6.6 Hz, 2-H), 1,54 (3H, d, J=6.9 Hz, 3-H); ¹³C NMR (75.5 MHz, DMSO-*d*₆)δ_{C} 160.4 (1-C), 44.1 (2-C), 19.8 (3-C); νₘₐₓ/cm⁻¹ 3388 (N-H), 2916, 2719, 2634, 2522 (tétrazole) ; MS(ESI) m/z 114.1 (M+H)⁺.

### 1.3. L-pyroglutamyl-L-1-aminoéthyltétrazole

Le L-pyroglutamyl-L-1-aminoéthyltétrazole (12), ou (*S*)-*N*-((*S*)-1-(1*H*-tétrazol-5-yl)éthyl)-5-oxopyrrolidine-2-carboxamide, peut être préparé selon une approche synthétique en phase solide, dans laquelle le point de départ est un L-1-aminoéthyltétrazole (6) fixé sur une résine de chlorure de 2-chloro-trityle (TP) et dont la fonction amine est protégée par un groupe protecteur Fmoc.

### a) Préparation du Fmoc-L-1-aminoethyltetrazole fixé sur résine (8)

L'approche utilisée est celle décrite par Chan et White ("*Fmoc solid phase peptide synthesis*", 2000). Le schéma réactionnel correspondant est présenté à la Figure 2.

La fonction amine du L-1-aminoéthyltétrazole (6) est protégée par un groupe protecteur Fmoc (fluorénylméthoxycarbonyle), en utilisant du chlorure de Fmoc en présence de Na₂CO₃, dans une réaction hétérogène en 2 phases. Ultérieurement, le Fmoc-L-1-aminoethyltetrazole (7) est attaché à la résine de chlorure de 2-chloro-trityle.

### b) Synthèse du L-pyroglutamyl-L-1-aminoéthyltétrazole (12)

Le schéma réactionnel correspondant est présenté à la Figure 3.

A l'étape (a), le groupe protecteur Fmoc du composé Fmoc-L-1-aminoéthyltétrazole (8), fixé à la résine, est éliminé avec de solution de pipéridine 25 % dans du DMF. A l'étape (b), la fonction amine libre est couplé avec Cbz-Pyr-OH en utilisant HBTU comme agent de couplage et DIPEA comme base non nucléophile dans le DMF. A l'étape (c), le Cbz-L-pyroglutamyl-L-1-aminoéthyltétrazole (10) attaché à la résine est clivé de la résine par un mélange TFA/DCM/TIS dans un rapport 5 : 95 : 1 équivalent. A l'étape (d), le Cbz-L-pyroglutamyl-L-1-aminoéthyltétrazole (11) est débarrassé de son groupe protecteur Cbz par une réaction d'hydrogénation catalysée au palladium.
Caractérisation : 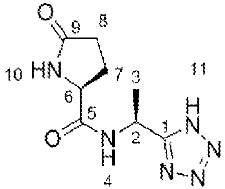 (*S*)-*N*-((*S*)-1-(1*H*-tétrazol-5-yl)éthyl)-5-oxopyrrolidine-2-carboxamide Masse molaire : 224,22 g/mol
T_{f}: 160°C (décomposé) ;
¹H NMR (300 MHz, D₂O) δ_{H} 5,35 (1H, quart, J=7.2 Hz, 2-H), 4.34 (1H, dd, J=4.8 Hz, J=8.4 Hz, 6-H), 2.52 (1H, m, 7-Hₐ), 2.40 (2H, m, 8-H) 2.04 (1H, m, 7-H_{b}), 1.61 (3H, d, J=6.9 Hz, 3-H); ¹³C NMR (75.5 MHz, D₂O) δ_{C} 182.3 (9-C), 174.6 (5-C), 159.0 (1-C), 56.9 (6-C), 41.0 (2-C), 29.3 (8-C), 25.0 (7-C), 18.1 (3-C); νₘₐₓ/cm⁻¹ 3262 (N-H), 1649 (C=O), 1547 (N-H); MS(ESI) m/z 225.1 (M+H)⁺

### EXEMPLE 2 : Evaluation de l'activité antibactérienne du L-pyroglutamyl-L-1-aminoéthyltétrazole (12)

Les concentrations minimales inhibitrices (CMI) du L-pyroglutamyl-L-1-aminoéthyltétrazole (12) pour 13 souches de bactéries à Gram négatif et 8 souches de bactéries à Gram positif ont été déterminées en utilisant un procédé de dilution en gélose décrit par Andrews JM. 2001 (« Détermination of minimum inhibitory concentrations » ; J. Antimicrob. Chemother. (2001) 48 Suppl. 1:5-16.) Celui-ci a nécessité l'utilisation d'un milieu sans antagoniste défini (sans peptone), préparé de la manière décrite précédemment avec l'inclusion de 2 % de sang de cheval lysé à la saponine, 25,0 µg/mL de NAD et 25,0 µg/mL d'hémine (Atherton et al., 1979 ; « Phosphonopeptides as antibacterial agents: rationale, chemistry, and structure-activity relationships » ; Antimicrob. Agents Chemother. (1979) 15:677-683). Le L-pyroglutamyl-L-1-aminoéthyltétrazole (12) a été dissous dans de l'eau désionisée stérile et incorporé dans le milieu gélosé à une plage de concentration de 1 à 128 µg/mL. Tous les isolats ont été préparés à une densité équivalente à 0,5 UFC dans de l'eau désionisée stérile à l'aide d'un densitomètre (environ 1,5 x 10⁸ UFC/mL), puis dilués à 1 pour 15. Une aliquote de 1 µL de chaque suspension diluée a été ensuite placée sur des plaques avec un inoculateur multipoint pour donner l'inoculation finale recommandée de 10 000 UFC/point (ou « spot » en langue anglaise) (Andrews, 2001. « Determination of minimum inhibitory concentrations» ; J. Antimicrob. Chemother. 48 Suppl 1:5-16). Toutes les boîtes (y compris les témoins sans antimicrobien) ont été incubées 22 heures à 37°C. Tous les essais ont été réalisés sur au moins deux réplicats indépendants afin d'examiner la reproductibilité.

Les isolats bactériens testés proviennent tous de collections internationales, l'*American Type Culture Collection* (ATCC) et la *National Collection of Type Cultures* (NCTC).

Les résultats de mesure de la CMI obtenus sont présentés dans le Tableau 2 ci-après.

**Tableau 2**

| | | Growth at various concentrations (mg/L) | | | | | | | | | CMI |
|---|---|---|---|---|---|---|---|---|---|---|---|
| | | 0 | 1 | 2 | 4 | 8 | 16 | 32 | 64 | 128 | (mg/L) |
| *Acinetobacter baumannii* | | + | + | + | + | + | + | + | + | + | > 128 |
| | ATCC 19606 | | | | | | | | | | |
| *Burkholderia cepacia* | | + | + | + | + | + | + | + | + | + | > 128 |
| | ATCC 25416 | | | | | | | | | | |
| *Enterobacter cloacae* | | + | + | + | + | + | + | + | + | + | > 128 |
| | NCTC 11936 | | | | | | | | | | |
| *Escherichia coli* | | + | + | + | + | + | + | + | + | + | > 128 |
| | NCTC 10418 | | | | | | | | | | |
| *Escherichia coli* | | + | + | + | + | + | + | + | + | + | > 128 |
| | NCTC 12241 | | | | | | | | | | |
| *Klebsiella pneumoniae* | | + | + | + | Tr. | - | - | - | - | - | 8 |
| | NCTC 9528 | | | | | | | | | | |
| *Providencia rettgeri* | | + | + | + | + | + | + | + | + | + | > 128 |
| | NCTC 7475 | | | | | | | | | | |
| *Pseudomonas aeruginosa* | | + | + | + | + | + | + | + | + | + | > 128 |
| | NCTC 10662 | | | | | | | | | | |
| *Salmonella* Typhimurium | | + | + | + | + | + | + | + | + | + | > 128 |
| | NCTC 74 | | | | | | | | | | |
| *Serratia marcescens* | | + | + | + | + | - | - | - | - | - | 8 |
| | NCTC 10211 | | | | | | | | | | |
| *Yersinia enterocolitica* | | + | + | Tr. | - | - | - | - | - | - | 4 |
| | NCTC 11176 | | | | | | | | | | |
| *Salmonella* Enteritidis | | + | + | + | + | + | + | + | + | + | > 128 |
| | NCTC 74 | | | | | | | | | | |
| *Salmonella* Enteritidis | | + | + | + | + | + | + | + | + | + | > 128 |
| | NCTC 6676 | | | | | | | | | | |
| *Bacillus subtilis* | | + | + | + | + | + | +/- | +/- | - | - | 64 |
| | NCTC 9372 | | | | | | | | | | |
| *Enterococcus faecalis* | | +/- | +/- | - | - | - | - | - | - | - | 2 |
| | NCTC 775 | | | | | | | | | | |
| *Enterococcus faecium* | | +/- | +/- | - | - | - | - | - | - | - | 2 |
| | NCTC 7171 | | | | | | | | | | |
| *Listeria monocytogenes* | | + | + | + | + | + | + | + | + | + | > 128 |
| | NCTC 11994 | | | | | | | | | | |
| *Staphylococcus epidermidis* | | + | + | + | + | + | + | + | + | + | > 128 |
| | NCTC 11047 | | | | | | | | | | |
| *Staphylococcus aureus* (MSSA) | | + | + | + | + | + | + | + | + | + | > 128 |
| | NCTC 6571 | | | | | | | | | | |
| *Staphylococcus aureus* (MRSA) | | + | + | + | + | + | + | + | + | + | > 128 |
| | NCTC 11939 | | | | | | | | | | |
| *Streptococcus agalactiae* | | + | + | + | + | + | + | + | + | + | > 128 |
| | NCTC 8181 | | | | | | | | | | |

D'après les données présentées, le L-pyroglutamyl-L-1-aminoéthyltétrazole (12) présente une activité antibactérienne plus ou moins prononcée selon l'espèce bactérienne considérée. Il est fortement inhibiteur vis-à-vis des souches de certaines espèces de bactéries, aussi bien à Gram négatif qu'à Gram positif, notamment *Klebsiella pneumoniae, Serratia marcescens, Yersinia enterocolitica, Bacillus subtilis, Enterococcus faecalis* et *Enterococcus faecium.* A des concentrations très faibles, de l'ordre de 2 mg/L, le L-pyroglutamyl-L-1-aminoéthyltétrazole (12) présente une excellente spécificité d'inhibition pour les entérocoques.

Les différences très significatives de concentration inhibitrice observées entre les différentes espèces de bactéries font du L-pyroglutamyl-L-1-aminoéthyltétrazole (12) un agent sélectif particulièrement bien adapté pour des milieux de culture microbiologique permettant la recherche, l'isolement et/ou l'enrichissement sélectifs de certaines espèces de bactéries, et plus particulièrement de bactéries appartenant aux espèces *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Listeria monocytogenes, Staphylococcus epidermidis, Staphylococcus aureus* et *Streptococcus agalactiae.*

## Revendications

1. L-pyroglutamyl-L-1-aminoéthyltétrazole, ayant pour formule développée :

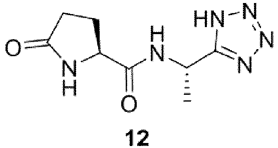

2. Utilisation *in vitro* du L-pyroglutamyl-L-1-aminoéthyltétrazole (12) conforme à la revendication 1, à titre de composé antibactérien.

3. Utilisation *in vitro* selon la revendication 2, dans une méthode ou un milieu de culture microbiologique destinés à la détection, l'identification, l'enrichissement, le dénombrement et/ou l'isolement de microorganismes.

4. Méthode de culture microbiologique *in vitro,* dans laquelle des microorganismes susceptibles d'être présents dans un échantillon à analyser et/ou à traiter sont ensemencés dans ou sur un milieu de culture microbiologique comprenant du L-pyroglutamyl-L-1-aminoéthyltétrazole (12) conforme à la revendication 1, à une concentration non nulle, égale ou inférieure à 1 g/L.

5. Méthode de culture selon la revendication 4, dans laquelle le L-pyroglutamyl-L-1-aminoéthyltétrazole est présent dans ledit milieu de culture microbiologique à une concentration :
- comprise entre 10 et 1000 mg/L, pour inhiber au moins une bactérie choisie parmi les espèces bactériennes consistant en : *Klebsiella pneumoniae, Serratia marcescens, Yersinia enterocolitica, Bacillus subtilis, Enterococcus faecalis* et *Enterococcus faecium* ; ou
- comprise entre 2 et 50 mg/L, pour inhiber au moins une bactérie choisie parmi les espèces bactériennes consistant en : *Klebsiella pneumoniae, Serratia marcescens, Yersinia enterocolitica, Enterococcus faecalis* et *Enterococcus faecium* ; ou
- comprise entre 1 et 10 mg/L, pour inhiber au moins une bactérie choisie parmi les espèces bactériennes consistant en : *Yersinia enterocolitica, Enterococcus faecalis* et *Enterococcus faecium* ; ou
- non nulle, égale ou inférieure à 5 mg/L, pour inhiber au moins une bactérie choisie parmi les espèces bactériennes consistant en : *Enterococcusfaecalis* et *Enterococcusfaecium ;*
ladite au moins une bactérie étant susceptible d'être présente dans ledit échantillon à analyser et/ou à traiter.

6. Méthode de culture selon la revendication 4 ou 5, dans laquelle, le L-pyroglutamyl-L-1-aminoéthyltétrazole est présent dans ledit milieu de culture microbiologique à une concentration :
- comprise entre 10 et 1000 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Listeria monocytogenes, Staphylococcus epidermidis, Staphylococcus aureus, Streptococcus agalactiae* ; ou
- comprise entre 2 et 50 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis, Listeria monocytogenes, Staphylococcus epidermidis, Staphylococcus aureus, Streptococcus agalactiae* ; ou
- comprise entre 1 et 10 mg/L, en vue de détecter, identifier, dénombrer et/ou isoler au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae* ; ou
- non nulle, égale ou inférieure à 5 mg/L, en vue de détecter, identifier, dénombrer et/ou isoler au moins un microorganisme cible choisi parmi les espèces bactériens consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae* ; ou
ledit au moins un microorganisme cible étant susceptible d'être présent dans ledit échantillon à analyser et/ou à traiter.

7. Milieu de culture microbiologique comprenant, à titre d'agent sélectif, au moins du L-pyroglutamyl-L-1-aminoéthyltétrazole (12) conforme à la revendication 1, à une concentration non nulle, égale ou inférieure à 1 g/L.

8. Milieu de culture microbiologique selon la revendication 7, pour la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae ;*
**caractérisé en ce que** le L-pyroglutamyl-L-1-aminoéthyltétrazole est présent à une concentration comprise entre 10 et 1000 mg/L.

9. Milieu de culture microbiologique selon la revendication 7, pour la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis, Listeria monocytogenes, Staphylococcus epidermidis, Staphylococcus aureus, Streptococcus agalactiae ;*
**caractérisé en ce que** le L-pyroglutamyl-L-1-aminoéthyltétrazole est présent à une concentration comprise entre 2 et 50 mg/L.

10. Milieu de culture microbiologique selon la revendication 7, pour la détection, l'identification, le dénombrement et/ou l'isolement d'au moins un microorganisme cible choisi parmi les espèces bactériennes consistant en : *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae ;*
**caractérisé en ce que** le L-pyroglutamyl-L-1-aminoéthyltétrazole est présent à une concentration non nulle, égale ou inférieure à 5 mg/L.

## Patentansprüche

1. L-Pyroglutamyl-L-1-aminoethyltetrazol mit der Strukturformel:

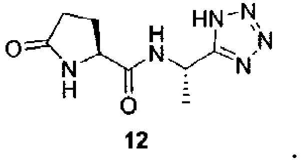

2. In-vitro-Verwendung von L-Pyroglutamyl-L-1-aminoethyltetrazol (12) nach Anspruch 1 als antibakterielle Verbindung.

3. In-vitro-Verwendung nach Anspruch 2 in einem mikrobiologischen Verfahren oder Kulturmedium zur Detektion, Identifizierung, Anreicherung, Zählung und/oder Isolierung von Mikroorganismen.

4. Verfahren zur mikrobiologischen In-vitro-Kultur, wobei Mikroorganismen, die in einer zu analysierenden und/oder zu behandelnden Probe vorhanden sein können, in oder auf einem mikrobiologischen Kulturmedium ausgesät werden, das L-Pyroglutamyl-L-1-aminoethyltetrazol (12) nach Anspruch 1 in einer Konzentration ungleich Null, gleich oder kleiner als 1 g/l umfasst.

5. Kulturverfahren nach Anspruch 4, wobei L-Pyroglutamyl-L-1-aminoethyltetrazol im mikrobiologischen Kulturmedium mit einer folgenden Konzentration vorhanden ist:
- zwischen 10 und 1000 mg/l, um mindestens eine Bakterie zu hemmen, die aus den Bakterienspezies ausgewählt ist, die aus: *Klebsiella pneumoniae, Serratia marcescens, Yersinia enterocolitica, Bacillus subtilis, Enterococcus faecalis* und *Enterococcus faecium* bestehen; oder
- zwischen 2 und 50 mg/l, um mindestens eine Bakterie zu hemmen, die aus den Bakterienspezies ausgewählt ist, die aus: *Klebsiella pneumoniae, Serratia marcescens, Yersinia enterocolitica, Enterococcus faecalis* und *Enterococcus faecium* bestehen; oder
- zwischen 1 und 10 mg/l, um mindestens eine Bakterie zu hemmen, die aus den Bakterienspezies ausgewählt ist, die aus: *Yersinia enterocolitica, Enterococcus faecalis* und *Enterococcus faecium* bestehen; oder
- ungleich Null, gleich oder kleiner als 5 mg/l, um mindestens eine Bakterie zu hemmen, die aus den Bakterienspezies ausgewählt ist, die aus: *Enterococcus faecalis* und *Enterococcus faecium* bestehen; wobei die mindestens eine Bakterie in der zu analysierenden und/oder zu behandelnden Probe vorhanden sein kann.

6. Kulturverfahren nach Anspruch 4 oder 5, wobei L-Pyroglutamyl-L-1-aminoethyltetrazol im mikrobiologischen Kulturmedium mit einer folgenden Konzentration vorhanden ist:
- zwischen 10 und 1000 mg/l zum Detektieren, Identifizieren, Zählen und/oder Isolieren mindestens eines Zielmikroorganismus, der aus den Bakterienspezies ausgewählt ist, die aus: *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Listeria monocytogenes, Staphylococcus epidermidis, Staphylococcus aureus, Streptococcus agalactiae* bestehen; oder
- zwischen 2 und 50 mg/l zum Detektieren, Identifizieren, Zählen und/oder Isolieren mindestens eines Zielmikroorganismus, der aus den Bakterienspezies ausgewählt ist, die aus: *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis, Listeria monocytogenes, Staphylococcus epidermidis, Staphylococcus aureus, Streptococcus agalactiae* bestehen; oder
- zwischen 1 und 10 mg/l zum Detektieren, Identifizieren, Zählen und/oder Isolieren mindestens eines Zielmikroorganismus, der aus den Bakterienspezies ausgewählt ist, die aus: *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae* bestehen; oder
- ungleich Null, gleich oder kleiner als 5 mg/l zum Detektieren, Identifizieren, Zählen und/oder Isolieren mindestens eines Zielmikroorganismus, der aus den Bakterienspezies ausgewählt ist, die aus *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae* bestehen; oder
wobei der mindestens eine Zielmikroorganismus in der zu analysierenden und/oder zu behandelnden Probe vorhanden sein kann.

7. Mikrobiologisches Kulturmedium, das als Selektionsmittel zumindest L-Pyroglutamyl-L-1-aminoethyltetrazol (12) nach Anspruch 1 in einer Konzentration ungleich Null, gleich oder kleiner als 1 mg/l umfasst.

8. Mikrobiologisches Kulturmedium nach Anspruch 7 zur Detektion, Identifizierung, Zählung und/oder Isolierung mindestens eines Zielmikroorganismus, der aus den Bakterienspezies ausgewählt ist, die aus: *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Yersinia enterocolitica, Salmonella* Enteritidis, *Bacillus subtilis, Listeria monocytogenes, Staphylococcus epidermidis, Staphylococcus aureus, Streptococcus agalactiae* bestehen;
**dadurch gekennzeichnet, dass** das L-Pyroglutamyl-L-1-aminoethyltetrazol in einer Konzentration zwischen 10 und 1000 mg/l vorhanden ist.

9. Mikrobiologisches Kulturmedium nach Anspruch 7 zur Detektion, Identifizierung, Zählung und/oder Isolierung mindestens eines Zielmikroorganismus, der aus den Bakterienspezies ausgewählt ist, die aus: *Acinetobacter baumannii, Burkholderia cepacia, Enterobacter cloacae, Escherichia coli, Providencia rettgeri, Pseudomonas aeruginosa, Salmonella* Typhimurium, *Salmonella* Enteritidis, *Bacillus subtilis, Listeria monocytogenes, Staphylococcus epidermidis, Staphylococcus aureus, Streptococcus agalactiae* bestehen;
**dadurch gekennzeichnet, dass** das L-Pyroglutamyl-L-1-aminoethyltetrazol in einer Konzentration zwischen 2 und 50 mg/l vorhanden ist.

10. Mikrobiologisches Kulturmedium nach Anspruch 7 zur Detektion, Identifizierung, Zählung und/oder Isolierung mindestens eines Zielmikroorganismus, der aus den Bakterienspezies ausgewählt ist, die aus: *Acinetobacter baumannii, Burkholderia cepacia, Enterobacter cloacae, Escherichia coli, Klebsiella pneumoniae, Providencia rettgeri, Pseudomonas aeruginosa, Salmonella* Typhimurium, *Serratia marcescens, Salmonella* Enteritidis, *Bacillus subtilis, Listeria monocytogenes, Staphylococcus epidermidis, Staphylococcus aureus, Streptococcus agalactiae* bestehen;
**dadurch gekennzeichnet, dass** das L-Pyroglutamyl-L-1-aminoethyltetrazol in einer Konzentration ungleich Null, gleich oder kleiner als 5 mg/l vorhanden ist.

## Claims

1. L-pyroglutamyl-L-1-aminoethyltetrazole having the developed formula:

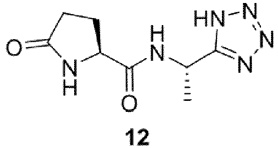

2. An *in vitro* use of L-pyroglutamyl-L-1-aminoethyltetrazole (12) as claimed in claim 1, as an antibacterial compound.

3. The *in vitro* use as claimed in claim 2, in a microbiological culture method or medium intended for the detection, identification, enrichment, counting and/or isolation of microorganisms.

4. An *in vitro* microbiological culture method, wherein microorganisms that may be present in a sample to be analyzed and/or to be treated are inoculated in or on a microbiological culture medium comprising L-pyroglutamyl-L-1-aminoethyltetrazole (12) as claimed in claim 1, at a non-zero concentration, less than or equal to 1 g/L.

5. The culture method as claimed in claim 4, wherein the L-pyroglutamyl-L-1-aminoethyltetrazole is present in said microbiological culture medium at a concentration:
- of between 10 and 1000 mg/L, for inhibiting at least one bacterium chosen from the bacterial species consisting of: *Klebsiella pneumoniae, Serratia marcescens, Yersinia enterocolitica, Bacillus subtilis, Enterococcus faecalis* and *Enterococcus faecium*; or
- of between 2 and 50 mg/L, for inhibiting at least one bacterium chosen from the bacterial species consisting of: *Klebsiella pneumoniae, Serratia marcescens, Yersinia enterocolitica, Enterococcus faecalis* and *Enterococcus faecium*; or
- of between 1 and 10 mg/L, for inhibiting at least one bacterium chosen from the bacterial species consisting of: *Yersinia enterocolitica, Enterococcus faecalis* and *Enterococcus faecium*; or
- which is non-zero, less than or equal to 5 mg/L, for inhibiting at least one bacterium chosen from the bacterial species consisting of: *Enterococcus faecalis* and *Enterococcus faecium*;
said at least one bacterium being capable of being present in said sample to be analyzed and/or to be treated.

6. The culture method as claimed in claim 4 or 5, wherein the L-pyroglutamyl-L-1-aminoethyltetrazole is present in said microbiological culture medium at a concentration:
- of between 10 and 1000 mg/L, with a view to detecting, identifying, counting and/or isolating at least one target microorganism chosen from the bacterial species consisting of: *Acinetobacter baumannii, Burkholderia cepacia, Enterobacter cloacae, Escherichia coli, Providencia rettgeri, Pseudomonas aeruginosa, Salmonella typhimurium, Salmonella enteritidis, Listeria monocytogenes, Staphylococcus epidermidis, Staphylococcus aureus, Streptococcus agalactiae*; or
- of between 2 and 50 mg/L, with a view to detecting, identifying, counting and/or isolating at least one target microorganism chosen from the bacterial species consisting of: *Acinetobacter baumannii, Burkholderia cepacia, Enterobacter cloacae, Escherichia coli, Providencia rettgeri, Pseudomonas aeruginosa, Salmonella typhimurium, Salmonella enteritidis, Bacillus subtilis, Listeria monocytogenes, Staphylococcus epidermidis, Staphylococcus aureus, Streptococcus agalactiae;* or
- of between 1 and 10 mg/L, with a view to detecting, identifying, counting and/or isolating at least one target microorganism chosen from the bacterial species consisting of: *Acinetobacter baumannii, Burkholderia cepacia, Enterobacter cloacae, Escherichia coli, Klebsiella pneumoniae, Providencia rettgeri, Pseudomonas aeruginosa, Salmonella typhimurium, Serratia marcescens, Salmonella enteritidis, Bacillus subtilis, Listeria monocytogenes, Staphylococcus epidermidis, Staphylococcus aureus, Streptococcus agalactiae*; or
- which is non-zero, less than or equal to 5 mg/L, with a view to detecting, identifying, counting and/or isolating at least one target microorganism chosen from the bacterial species consisting of: *Acinetobacter baumannii, Burkholderia cepacia, Enterobacter cloacae, Escherichia coli, Klebsiella pneumoniae, Providencia rettgeri, Pseudomonas aeruginosa, Salmonella typhimurium, Serratia marcescens, Yersinia enterocolitica, Salmonella enteritidis, Bacillus subtilis, Listeria monocytogenes, Staphylococcus epidermidis, Staphylococcus aureus, Streptococcus agalactiae*; or
said at least one target microorganism being capable of being present in said sample to be analyzed and/or to be treated.

7. A microbiological culture medium comprising, as selective agent, at least L-pyroglutamyl-L-1-aminoethyltetrazole (12) as claimed in claim 1, at a non-zero concentration, less than or equal to 1 g/L.

8. The microbiological culture medium as claimed in claim 7, for the detection, identification, counting and/or isolation of at least one target microorganism chosen from the bacterial species consisting of: *Acinetobacter baumannii, Burkholderia cepacia, Enterobacter cloacae, Escherichia coli, Klebsiella pneumoniae, Providencia rettgeri, Pseudomonas aeruginosa, Salmonella typhimurium, Serratia marcescens, Yersinia enterocolitica, Salmonella enteritidis, Bacillus subtilis, Listeria monocytogenes, Staphylococcus epidermidis, Staphylococcus aureus, Streptococcus agalactiae;*
**characterized in that** the L-pyroglutamyl-L-1-aminoethyltetrazole is present at a concentration of between 10 and 1000 mg/L.

9. The microbiological culture medium as claimed in claim 7, for the detection, identification, counting and/or isolation of at least one target microorganism chosen from the bacterial species consisting of: *Acinetobacter baumannii, Burkholderia cepacia, Enterobacter cloacae, Escherichia coli, Providencia rettgeri, Pseudomonas aeruginosa, Salmonella typhimurium, Salmonella enteritidis, Bacillus subtilis, Listeria monocytogenes, Staphylococcus epidermidis, Staphylococcus aureus, Streptococcus agalactiae;*
**characterized in that** the L-pyroglutamyl-L-1-aminoethyltetrazole is present at a concentration of between 2 and 50 mg/L.

10. The microbiological culture medium as claimed in claim 7, for the detection, identification, counting and/or isolation of at least one target microorganism chosen for the bacterial species consisting of: *Acinetobacter baumannii, Burkholderia cepacia, Enterobacter cloacae, Escherichia coli, Klebsiella pneumoniae, Providencia rettgeri, Pseudomonas aeruginosa, Salmonella typhimurium, Serratia marcescens, Salmonella enteritidis, Bacillus subtilis, Listeria monocytogenes, Staphylococcus epidermidis, Staphylococcus aureus, Streptococcus agalactiae;*
**characterized in that** the L-pyroglutamyl-L-1-aminoethyltetrazole is present at a non-zero concentration, less than or equal to 5 mg/L.
